# EUROPÄISCHE PATENTSCHRIFT

(11) **EP 3 518 814 B1**
(45) Veröffentlichungstag und Bekanntmachung des Hinweises auf die Patenterteilung: **29.07.2020**
(21) Anmeldenummer: 17780064.6
(22) Anmeldetag: 28.09.2017
(51) Int. Cl.: A61C 1/14

(54) **ZAHNÄRZTLICHES BEHANDLUNGSINSTRUMENT ZUM BETREIBEN EINES ROTATIONSWERKZEUGS**
DENTAL TREATMENT INSTRUMENT FOR OPERATING A ROTATING TOOL
INSTRUMENT DENTAIRE DE TRAITEMENT POUR LE FONCTIONNEMENT D'UN OUTIL ROTATIF

(30) Priorität: 28.09.2016 EP 16191082
(43) Veröffentlichungstag der Anmeldung: 07.08.2019
(73) Patentinhaber: KaVo Dental GmbH, 88400 Biberach (DE)
(72) Erfinder: BUTSCHER, Jürgen, 88299 Leutkirch (DE); CLASSEN, Thomas, 88518 Herbertingen (DE)
(74) Vertreter: Thun, Clemens
(86) Internationale Anmeldenummer: PCT/EP2017/074651
(87) Internationale Veröffentlichungsnummer: WO 2018/060343

(56) Entgegenhaltungen:
- DE-A1-102004 007 413
- JP-A- 2010 220 635
- US-A1- 2008 243 123
- US-A1- 2014 163 555

## Beschreibung

Die Erfindung betrifft ein zahnärztliches Behandlungsinstrument zum Betreiben eines Rotationswerkzeugs.

Aus dem Stand der Technik ist es bekannt, mit einem zahnärztlichen Behandlungsinstrument und einem Rotationswerkzeug eine (natürliche oder künstliche) Zahnkrone aufzutrennen. Dabei ist es in der zahnärztlichen Praxis regelmäßig erwünscht, den hierbei erforderlichen zeitlichen Aufwand möglichst gering zu halten. Um einen entsprechenden Arbeitsprozess geeignet zu ermöglichen, gibt es auf dem Werkzeugmarkt diverse Werkzeuge, die auf die verschiedenen Werkstoffeigenschaften von künstlichen Kronen abgestimmt sind. Die Kronen werden in folgende Typen eingeteilt:
- Metallkrone (Goldkrone, Titan oder Stahl)
- Verblendkrone (Metallgerüst, Keramikverblendung)
- Vollkeramikkrone (Zirkon, Feldspat-, Glas- oder Alumiumoxidkeramik)
- Kunststoffkrone

Die Rotationswerkzeuge unterscheiden sich hierbei durch die auf das jeweilige Material abgestimmten Schneiden-Geometrien, die verwendeten Werkstoffe und die Beschichtungen. Die bekannten Rotationswerkzeuge weisen allesamt den als solchen üblichen, zylindrischen Schaft nach der Norm DIN EN ISO 1797 (insbesondere DIN EN ISO 1797-1 und DIN EN ISO 1797-2) auf.

Die von den Herstellern empfohlenen Drehzahlen zum Antrieb von Dentalwerkzeugen sind ausschließlich durch hochtourig laufende Instrumente, wie Schnelllaufwinkelstücke und Turbinen erreichbar. In diesen Instrumenten sind kraftschlüssige Spannelemente verbaut, die das Werkzeug sowohl im Stillstand, als auch im Betriebszustand in Position halten, um ein unbeabsichtigtes Lösen und dadurch eine Gefährdung für Patient, Behandler oder Dritte zu vermeiden.

Die auftretenden Drehmomente an der Schneide des Werkzeugs werden allein durch die kraftschlüssige Verbindung und die durch die Oberflächengüten vorherrschenden Reibmomente aufgenommen. Denkbar sind auch formschlüssige Verbindungen.

In Abhängigkeit der gewählten Ausführung der Werkzeugschneide, des zu bearbeitenden Kronenmaterials, der Geometrie der Krone und der eingestellten Drehzahl des Instruments kann es während eines Arbeitsvorgangs mit dem Werkzeug zu ruckartigen, schlagenden und reißenden auf das Spannelement wirkenden Impulsen kommen. Dies kann bereits bei geringen Spitzenlasten zu einem Durchrutschen des Werkzeugschafts bzw. zu einem Schlupf im Spannbereich führen und die Oberfläche des Schaftes beschädigen.

In der täglichen Praxis werden die Vorgaben der Werkzeug- und Instrumentenhersteller, nur glatte und unbeschädigte Schäfte zu verwenden, oft missachtet und die Werkzeuge so lange verwendet, bis die Schneide ihre Funktion verliert. Hierdurch ist die Gefahr eines Qualitätsverlustes der Instrumente bzw. der Spannsysteme gegeben. Wie aus Marktbeobachtungen und Kundenbefragungen bekannt, kommt es in der Praxis zu folgenden Problemen: Überhitzung von Instrumenten aufgrund sich ergebender Reibwärme durch Schlupf, klemmende Werkzeuge oder nur durch erhöhten Kraftaufwand aus dem Behandlungsinstrument zu entnehmende Werkzeuge bis hin zu fehlender bzw. unzureichender Haltekraft des Spannelements. Dies wirkt sich insbesondere grundsätzlich auch auf die Lebenszeit der betreffenden Behandlungsinstrumente, also beispielsweise der Winkelstücke aus.

Bei Schnelllaufwinkelstücken und Turbinen sind ein leises und angenehmes Laufgeräusch, ein geringer Bohrerschlag und die Produktlebensdauer wichtige Qualitätsmerkmale, die ganz wesentlich durch die Qualität der einzelnen Bauteile und Baugruppen beeinflusst werden. Insbesondere die Qualität der rotierenden Teile, wie Kopftrieb bzw. Turbinenläufer, wirkt sich stark auf die Qualität des Gesamtproduktes aus. Durch hohe Drehfrequenzen dieser Baugruppen können sich selbst kleine Abweichungen der Form von Bauteilen vom Idealzustand, wie Unrundheiten, Planschläge, Radialschläge oder Abweichungen mit Bezug auf die Koaxialität signifikant auf die oben beschriebenen Qualitätsmerkmale des Gesamtproduktes auswirken.

Schnelllaufwinkelstücke und Turbinen besitzen im Allgemeinen mit Bezug auf die Fixierung eines eingesetzten Werkzeugs einen vergleichbaren konstruktiven Aufbau. Dieser umfasst ein Spannelement, um das Werkzeug in Position zu halten und mindestens einen Führungsbereich, um das Werkzeug zu führen. Zudem gibt es ein meist axial bewegliches Element, um das Spannelement über eine manuelle Kraft bedienen zu können. Zur Führung dieser Teile dient eine Lager- bzw. Antriebshülse bzw. ein so genannter Kopftrieb für Schnelllaufwinkelstücke oder eine Achse für Turbinen. Um Werkzeuge mit glatten Schäften dauerhaft sicher führen und halten zu können, werden im Spannsystem meist Bauteile aus hochwertigen, härtbaren Edelstählen oder Edelstählen mit einer Hartschicht - oft sogar aus Hartmetall - verbaut, die bereits in der Lagerhülse integriert sind, eingepresst und/oder miteinander verschweißt sind. Keramische Werkstoffe finden hier ebenfalls Anwendung.

Aus der DE 10 2004 007 413 A1 ist ein dentalmedizinisches Handstück mit einem Handstückkopf bekannt, in dem eine antreibbare Aufnahmehülse drehbar gelagert ist, in die sich ein Werkzeug mit seinem Schaftbereich einstecken lässt. Zwischen der Aufnahmehülse und dem Werkzeugschaft wirkt zur Übertragung eines Drehmoments eine Zahnkupplung. Zur Verhinderung des Rausrutschens des Werkzeugschafts aus der Aufnahmehülse wirkt eine Axialkupplung mit einer Kugel als Kupplungselement, die hierzu in ein am Werkzeugschaft ausgebildetes Kupplungsloch eingreift, wodurch eine punktuelle Spannung zwischen der Kugel und dem Werkzeugschaft erzielt wird.

Der Erfindung liegt die Aufgabe zu Grunde, ein zahnärztliches Behandlungsinstrument zum Betreiben eines Rotationswerkzeugs anzugeben, das mit Bezug auf die oben skizzierten Nachteile vorteilhafte Eigenschaften aufweist. Insbesondere soll sich das Behandlungsinstrument dazu eignen, besonders hohen Anforderungen, wie sie beispielsweise im Rahmen eines Auftrennens von Kronen gegeben sind, gerecht werden zu können.

Diese Aufgabe wird gemäß der Erfindung mit dem in dem unabhängigen Anspruch genannten Gegenstand gelöst. Besondere Ausführungsarten der Erfindung sind in den abhängigen Ansprüchen angegeben.

Gemäß der Erfindung ist ein zahnärztliches Behandlungsinstrument zum Betreiben eines Rotationswerkzeugs vorgesehen, wobei das Behandlungsinstrument einen zur Kopplung mit einem Schaft des Rotationswerkzeugs vorgesehenen Kopftrieb mit einer hülsenartigen Führungsbuchse aufweist. Zur Fixierung des in die Führungsbuchse eingesetzten Rotationswerkzeugs weist Letztere eine Spannzange auf. Dabei weist die Führungsbuchse in zumindest einem ersten axialen Bereich Flächenabschnitte auf, welche Teile einer Zylinderform bilden, wobei von diesen Flächenabschnitten radial nach außen weisende Ausnehmungen vorgesehen sind, welche der Innenkontur der Führungsbuchse eine von einer rotationssymmetrischen Form abweichende Form verleihen.

Durch die Flächenabschnitte, die Teile einer Zylinderform bilden, lässt sich eine besonders geeignete Führung bzw. Abstützung eines kreiszylindrischen Schaftes eines Rotationswerkzeugs bewirken. Außerdem eignen sich die Ausnehmungen zum Eingriff von radialen Fortsätzen eines nicht-kreiszylindrischen Schaftabschnittes eines Rotationswerkzeugs, so dass sich ein derartiger nicht-kreiszylindrischer Schaftabschnitt besonders geeignet mit einer formschlüssigen Kontur antreiben lässt. Somit bietet das erfindungsgemäße Behandlungsinstrument zum einen die Möglichkeit, Werkzeugschäfte mit einer entsprechend "geänderten" Schaftgeometrie aufzunehmen, um entsprechenden, besonders hohen Anforderungen gerecht zu werden und den genannten Nachteilen und Schwachpunkten eines Schafts nach DIN EN ISO 1797 entgegenzuwirken und zum anderen die Möglichkeit, für Standardanwendungen weiterhin die Werkzeuge nach DIN EN ISO 1797 verwenden zu können.

Vorzugsweise weist die Führungsbuchse dabei einen zweiten axialen Bereich auf, dessen Innenkontur eine vollständige Zylinderform bildet, welche hinsichtlich ihres Durchmessers identisch zu der durch die Flächenabschnitte des ersten axialen Bereichs gebildeten Zylinderform ist. Dies ist vorteilhaft mit Bezug auf eine möglichst geeignete Führung des kreiszylindrischen Schaftes entlang seiner Längsachse.

Vorzugsweise umfassen Abschnitte des zweiten axialen Bereichs die Spannzange. So lässt sich ein rein zylindrischer Schaft eines Rotationswerkzeugs besonders geeignet durch die Spannzange in seiner Lage fixieren. Insbesondere kann hierzu die Gestaltung derart sein, dass die Innenkontur des zweiten axialen Bereichs teilweise durch die Spannzange gebildet ist.

Vorzugsweise schließt sich der erste axiale Bereich an eine Einführungsöffnung für das Werkzeug an. Auf diese Weise lässt sich ein Rotationswerkzeug mit einem entsprechenden nicht-kreiszylindrischen Schaft besonders einfach in die Führungsbuchse einbringen, insbesondere, wenn der Schaft radiale Eingriffselemente zum Eingriff in die Ausnehmungen aufweist, die fest mit dem restlichen Schaft verbunden sind.

Vorzugsweise ist eine bzw. die Länge des ersten axialen Bereichs kleiner ist als eine bzw. die Länge des zweiten axialen Bereichs. Hierdurch lässt sich eine besonders geeignete Führung eines Werkzeugschaftes erzielen; beispielsweise kann vorgesehen sein, dass die Länge des ersten axialen Bereichs höchstens 20% der Länge des zweiten axialen Bereichs beträgt.

Vorzugsweise weist die Führungsbuchse eine Lager- bzw. Antriebshülse auf, welche in einem Kopfbereich des Behandlungsinstruments drehbar gelagert ist und mit Antriebsmitteln des Behandlungsinstruments zusammenwirkt. So lässt sich eine besonders effektive Drehmomentübertragung auf den Schaft des Werkzeugs bewirken. Dabei ist weiterhin vorzugsweise der erste axiale Bereich der Führungsbuchse als integraler Bestandteil der Antriebshülse gestaltet. Hierdurch ist eine besonders unmittelbare Drehmomentübertragung ermöglicht.

Vorzugsweise besteht die Antriebshülse aus einem härteren Material als die Spannzange. Dies ist vorteilhaft mit Bezug auf die Eignung der Antriebshülse zur Drehmomentübertragung und die Eignung der Spannzange zur Fixierung des Rotationswerkzeugs.

Vorzugsweise bilden die Ausnehmungen längliche Kerben, welche sich axial erstrecken. Dabei sind weiterhin vorzugsweise mehrere Kerben vorgesehen, welche am Umfang der Rotationsform verteilt, vorzugsweise gleichmäßig verteilt angeordnet sind. Dies ist herstellungstechnisch vorteilhaft und ermöglicht dabei eine geeignete Drehmomentübertragung.

Vorzugsweise weist das Behandlungsinstrument Antriebsmittel zum Übertragen einer Rotation auf ein in die Führungsbuchse aufgenommenes Rotationswerkzeug auf. Insbesondere können diese Antriebsmittel einen Elektromotor oder eine Turbine oder eine Luftversorgung/Druckluft umfassen. Die Antriebsmittel können elektrischer oder fluider Art sein. Beispielsweise können die Antriebsmittel einen Luftmotor umfassen.

Vorzugsweise ist die Spannzange in eine radiale Ausnehmung der Antriebshülse eingesetzt. Dies ist bei geeigneter Herstellungsmöglichkeit vorteilhaft mit Bezug auf die Drehmomentübertragung von der Antriebshülse auf die Spannzange und im Weiteren auf das Werkzeug.

Vorzugsweise ist die Spannzange mit Bezug auf die Längsachse auf einer Seite des ersten axialen Bereichs angeordnet, die der Einführungsöffnung gegenüber liegt. So eignet sich die Führungsbuchse zur Fixierung eines Werkzeugs, das einen zylindrischen Schaftabschnitt aufweist, an den sich ein weiterer Schaftabschnitt anschließt, der zur formschlüssigen Wechselwirkung mit dem ersten axialen Bereich gestaltet ist.

Vorzugsweise ist der erste axiale Bereich der Führungsbuchse mit Hilfe eines separaten Bauteils gestaltet, das in die Antriebshülse eingesetzt ist. Dies ist herstellungstechnisch vorteilhaft. Dabei ist weiterhin vorzugsweise die Spannzange mit Bezug auf die Längsachse auf einer Seite des separaten Bauteils angeordnet, die der Einführungsöffnung gegenüber liegt. Dies ist wiederum vorteilhaft mit Bezug auf die Fixierungsmöglichkeit eines Werkzeugs mit einem zylindrischen Schaftabschnitt und einem sich an diesen anschließenden weiteren Schaftabschnitt zur formschlüssigen Wechselwirkung mit dem ersten axialen Bereich.

Die Erfindung wird im Folgenden anhand eines Ausführungsbeispiels und mit Bezug auf die Zeichnungen näher erläutert. Es zeigen:
- Fig. 1a: eine schematische Skizze einer Seitenansicht eines Kopfbereichs eines anmeldungsgemäßen Behandlungsinstruments, wobei ein Bereich einer Führungsbuchse, die zur Aufnahme eines Werkzeugschaftes dient, nach Art eines Querschnitts gezeigt ist.
- Fig. 1b: eine der Fig. 1a entsprechende Querschnittskizze,
- Fig. 2: einen vergrößerten Ausschnitt aus Fig. 1a,
- Fig. 3: eine Skizze eines Schnitts der Führungsbuchse längs der in Fig. 1a bezeichneten Linie III - III,
- Fig. 4a: eine, der Darstellung der Fig. 1a entsprechende Darstellung zu einer Variante, bei der ein erster axialer Bereich der Führungsbuchse als ein integraler Bestandteil einer Spannzange gestaltet ist,
- Fig. 4b: eine der Fig. 4a entsprechende Querschnittskizze,
- Fig. 5: eine, der Darstellung der Fig. 4a entsprechende Darstellung zu einer weiteren Variante, bei der der erste axiale Bereich der Führungsbuchse mit Hilfe eines eigenständigen Bauteils gestaltet ist,
- Fig. 6: eine perspektivische Schnittskizze des in Fig. 5 gezeigten Bauteils,
- Fig. 7: eine Ansicht des in Fig. 5 gezeigten Bauteils entlang der Längsachse,
- Fig. 8: eine perspektivische Schnittskizze zu einer Variation des in Fig. 5 gezeigten Bauteils und
- Fig. 9: eine Ansicht der in Fig. 8 gezeigten Variation des Bauteils entlang der Längsachse.

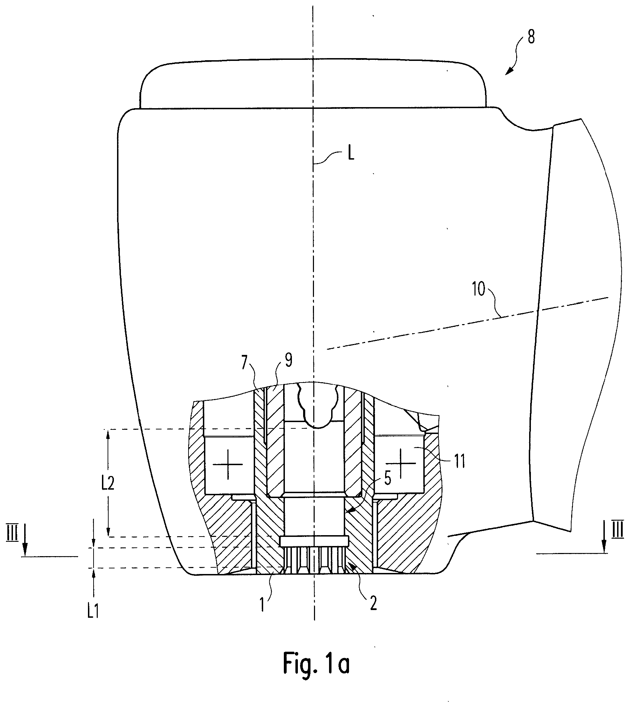

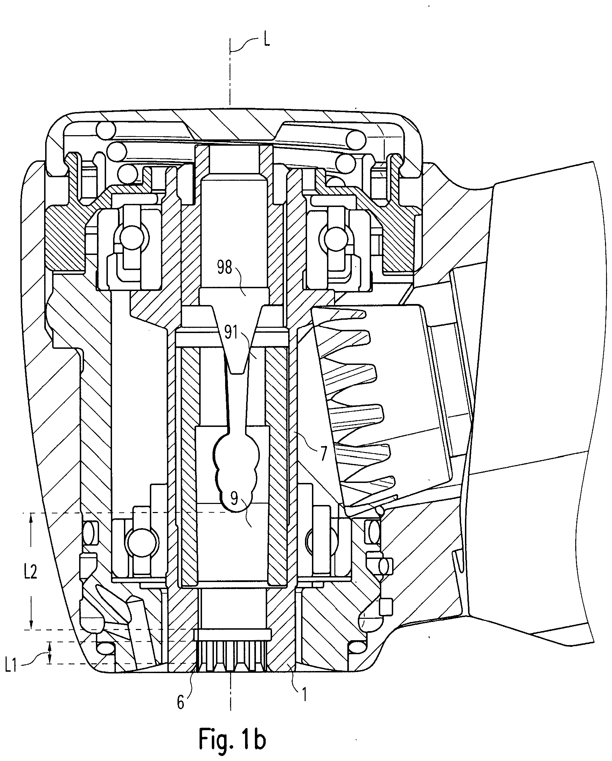

Fig. 1a zeigt eine schematische Skizze einer Seitenansicht eines Kopfbereichs 8 eines anmeldungsgemäßen zahnärztlichen Behandlungsinstruments zum Betreiben eines Rotationswerkzeugs. Fig. 1b zeigt eine entsprechende Querschnittskizze. Bei dem Behandlungsinstrument kann es sich beispielsweise um ein Winkelstück, insbesondere ein Schnelllaufwinkelstück oder um eine Turbine handeln.

Bei dem Rotationswerkzeug, hier im Folgenden auch kurz als Werkzeug bezeichnet, kann es sich beispielsweise um einen Bohrer handeln. Insbesondere weist das Werkzeug einen Schaftbereich oder kurz Schaft auf, der dazu vorgesehen ist, in den Kopfbereich 8 des Behandlungsinstruments eingesteckt zu werden.

Das Behandlungsinstrument kann - wie an sich bekannt und üblich - einen Handgriffteil aufweisen, der sich an den Kopfbereich 8 anschließt und der zum Halten des Behandlungsinstruments vorgesehen ist. In Fig. 1a ist lediglich eine Hauptachse 10 des Handgriffteils angedeutet.

Das Behandlungsinstrument weist einen zur Kopplung mit dem Schaft des Werkzeugs vorgesehenen, insbesondere in dem Kopfbereich 8 angeordneten Kopftrieb mit einer hülsenartigen Führungsbuchse 1 auf. Insbesondere ist die Führungsbuchse 1 derart gestaltet, dass der Schaft des Werkzeugs zur Kopplung in die Führungsbuchse 1 eingesteckt werden kann. Durch die Führungsbuchse 1 ist eine Längsachse L festgelegt. Die Gestaltung ist dabei derart, dass der Schaft des Werkzeugs mit seiner Schaft-Achse parallel zu der Längsachse L ausgerichtet ist, wenn das Werkzeug wie vorgesehen mit dem Behandlungsinstrument verbunden ist, also in die Führungsbuchse 1 eingesetzt ist.

Wie in Fig. 1b skizziert, weist die Führungsbuchse 1 eine Spannzange 9 zur Fixierung eines in die Führungsbuchse 1 aufgenommenen Rotationswerkzeugs auf. Insbesondere ist die Spannzange 9 dazu ausgestaltet, das Werkzeug sowohl axial zu sichern, als auch derart zu fixieren, dass eine für einen Betrieb des Behandlungsinstruments geeignete Übertragung des Drehmoments von der Führungsbuchse 1 auf das Werkzeug ermöglicht ist.

Insbesondere ist die Gestaltung derart, dass die Führungsbuchse 1 drehbar, beispielsweise mittels eines Drehlagers 11 drehbar in dem Kopfbereich 8 des Behandlungsinstruments gelagert ist und dazu ausgestaltet ist, ein Drehmoment auf den Schaft des Werkzeugs zu übertragen, um das Werkzeug auf diese Weise anzutreiben. Vorzugsweise weist das Behandlungsinstrument hierzu Antriebsmittel zum Übertragen des Drehmoments bzw. einer Rotation auf das in die Führungsbuchse 1 aufgenommene Werkzeug auf. Die Antriebsmittel können dabei insbesondere einen Elektromotor oder eine Turbine umfassen.

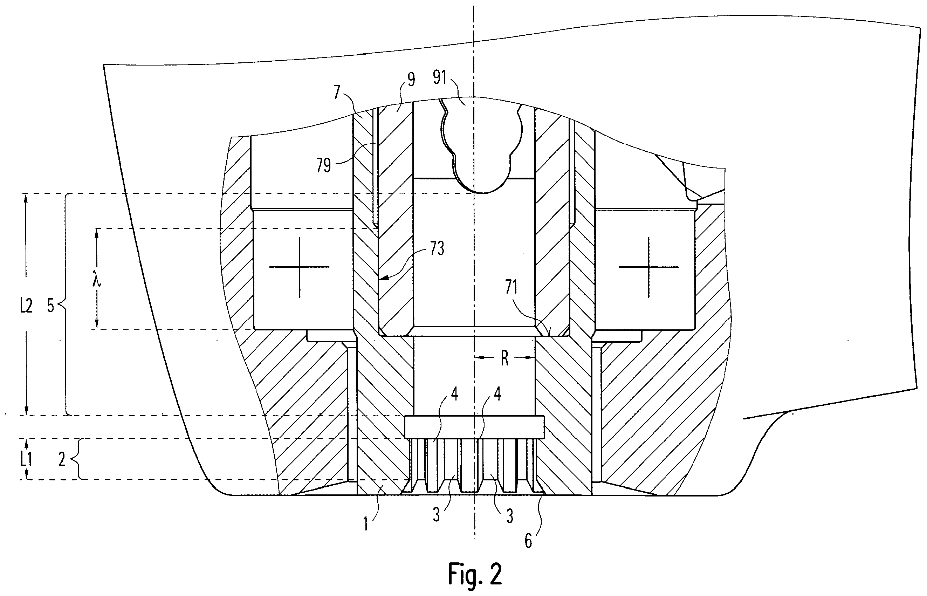

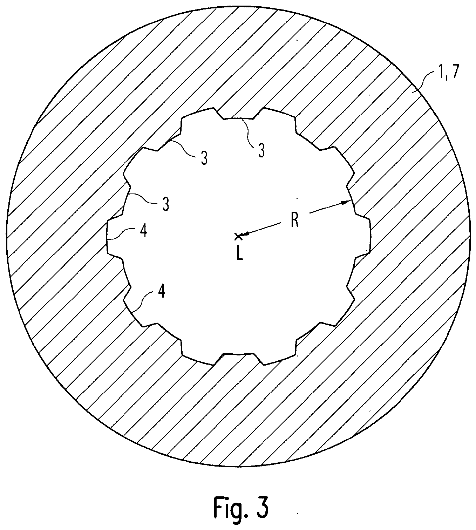

Fig. 2 zeigt einen vergrößerten Ausschnitt aus Fig. 1a und in Fig. 3 ist eine Querschnitt-Skizze der Führungsbuchse 1 normal zu der Längsachse L gezeigt, und zwar auf der in Fig. 1a mit III - III bezeichneten Höhe. Die Führungsbuchse 1 weist entlang der Längsachse L betrachtet in zumindest einem ersten Längsabschnitt 2, hier als erster axialer Bereich 2 bezeichnet, Flächenabschnitte 3 auf, welche Teile einer Zylinderform bilden, insbesondere einer kreisförmigen Zylinderform, die sich mit ihrer Symmetrieachse parallel zu der Längsachse L erstreckt. In Fig. 3 bezeichnet das Bezugszeichen L die Lage der Längsachse und mit dem Bezugszeichen R ist der Radius der genannten Zylinderform bezeichnet. Der Durchmesser der Zylinderform beträgt dementsprechend 2R. Vorzugsweise ist die Gestaltung derart, dass - in einem Querschnitt normal zur Längsachse L betrachtet - die Flächenabschnitte 3 jeweils einen Kreisbogen beschreiben, dessen Mittelpunktwinkel mindestens 5°, besonders bevorzugt mindestens 10° beträgt. Dies ist vorteilhaft, weil sich hierdurch ein Werkzeug mit einem rein zylindrischen Schaftabschnitt, dessen Radius R beträgt, durch die Flächenabschnitte 3 besonders geeignet radial sichern lässt.

Wie weiterhin in Fig. 3 beispielhaft skizziert, sind die Flächenabschnitte 3 vorzugsweise mit Bezug auf den Umfang der Zylinderform gleichmäßig verteilt ausgebildet. Im gezeigten Beispiel beträgt die Anzahl der Flächenabschnitte 3 zehn. Die Anzahl kann beispielsweise zwischen zwei und dreißig betragen, vorzugsweise zwischen vier und zwanzig.

Vorzugsweise ist die Gestaltung derart, dass die Flächenabschnitte 3 jeweils eine längliche Form aufweisen, wobei sie sich insbesondere jeweils parallel zur Längsachse L erstrecken.

Von den Flächenabschnitten 3 sind mit Bezug auf die Längsachse L radial nach außen weisende Ausnehmungen 4 vorgesehen bzw. ausgebildet, welche der Innenkontur der Führungsbuchse 1 eine von einer rotationssymmetrischen bzw. kreiszylinderförmigen Form abweichende Form verleihen.

Ein Werkzeug mit einem Schaft, der radiale Eingriffselemente aufweist, die zu den Ausnehmungen 4 korrespondieren, eignet sich daher besonders zur Drehmomentübertragung, denn in diesem Fall lässt sich eine entsprechende formschlüssige Verbindung zwischen dem Werkzeug und der Führungsbuchse 1 herstellen. Unter Nutzung eines derartigen Werkzeugs eignet sich das Behandlungsinstrument daher insbesondere zur Übertragung besonders hoher Drehmomente, wie sie beispielsweise im Rahmen eines Auftrennens einer Krone typischerweise auftreten können. Insbesondere eignet sich die Führungsbuchse 1 zur Fixierung eines Werkzeugs mit einem Schaft, der in einem ersten Schaftbereich rein kreiszylindrisch geformt ist, insbesondere mit dem Radius R und in einem zweiten Schaftbereich die genannten radialen Eingriffselemente aufweist, deren radiale Erstreckung dementsprechend größer als R ist.

Allerdings eignet sich das Behandlungsinstrument nicht nur zum Betreiben eines Werkzeugs mit einem Schaft, der entsprechende Eingriffselemente aufweist, sondern alternativ auch zum Betreiben eines Rotationswerkzeugs mit einem rein zylindrischen Schaftabschnitt bzw. einem Schaft gemäß der eingangs genannten Norm DIN EN ISO 1797, denn ein solcher rein zylindrischer Schaft bzw. Schaftabschnitt lässt sich durch die Spannzange entsprechend geeignet fixieren.

Vorzugsweise ist die Gestaltung derart, dass die Ausnehmungen 4 jeweils eine längliche Form aufweisen, wobei sie sich insbesondere jeweils parallel zur Längsachse L erstrecken. Beispielsweise können die Ausnehmungen 4 längliche Kerben bilden bzw. durch längliche Kerben gebildet sein. Dabei sind vorzugsweise mehrere Kerben vorgesehen, welche am Umfang der Rotationsform verteilt, vorzugsweise gleichmäßig verteilt angeordnet sind. Vorzugsweise ist die Anzahl der Ausnehmungen 4 gleich der Anzahl der Flächenabschnitte 3.

Die formschlüssige Kontur kann dabei beispielsweise als Feder- oder Profilwellenverbindung, wie z. B. einer Scheiben-, Federverbindung oder Keilwellenverbindung bzw. Zahnwellenverbindung ausgeführt sein. Formschlüssige Verbindungen sind lösbare Verbindungen und unterscheiden sich in ihren Anwendungsbereichen. Treten große Kerbspannungen auf, so werden Feder- oder Keilwellenverbindungen mit parallelen Flanken bevorzugt. Federverbindungen haben jedoch den Nachteil, dass bei großen Drehzahlen eine ungeeignete Unwucht entstehen kann. Für geringe Kerbspannungen bieten sich Zahnwellenverbindungen bzw. Kerbzahnwellenverbindungen an, die u. a. für geringe Profilhöhen geeignet sind. Sie können herstellungstechnisch vorteilhaft zum Beispiel mittels Prägen hergestellt werden. Etwaig auftretende Spitzenlasten an der formschlüssigen Verbindung werden auf die Getriebekomponenten verteilt und könnten gegebenenfalls über eine Rutschkupplung egalisiert werden.

Die beschriebene Innenkontur der Führungsbuchse 1 kann herstellungstechnisch vorteilhaft beispielsweise mittels Räumen, Stoßen oder Prägen eingearbeitet sein.

Vorzugsweise ist die Gestaltung derart, dass die Innenkontur der Führungsbuchse 1 über den ersten axialen Bereich 2 hinweg betrachtet keinen Teil aufweist, der zur Längsachse L einen Abstand aufweist, der kleiner als der Radius R der Zylinderform ist. Insbesondere ist die Gestaltung derart, dass die Innenkontur der Führungsbuchse 1 über den ersten axialen Bereich 2 hinweg betrachtet lediglich durch die Flächenabschnitte 3 und die Ausnehmungen 4 gebildet ist. Hierdurch eignet sich die Führungsbuchse 1 besonders zur Aufnahme eines kreiszylinderförmigen Werkzeugschaftes mit dem Durchmesser 2R. Vorzugsweise ist der Durchmesser 2R derart gewählt, dass sich die Führungsbuchse 1 zur Aufnahme eines Werkzeugschaftes eignet, der der eingangs genannten Norm DIN EN ISO 1797 entspricht.

Wie im gezeigten Beispiel der Fall und in Fig. 1a angedeutet, weist die Führungsbuchse 1 außerdem entlang der Längsachse L betrachtet einen zweiten Längsabschnitt 5 auf, hier als zweiter axialer Bereich 5 bezeichnet, dessen Innenkontur eine vollständige Zylinderform bildet. Dabei ist der Durchmesser dieser vollständigen Zylinderform gleich dem Durchmesser 2R der durch die Flächenabschnitte 3 des ersten axialen Bereichs 2 gebildeten Zylinderform. So eignet sich dieser zweite axiale Bereich 5 besonders zur Führung eines entsprechenden Schaftes.

Insbesondere kann die Führungsbuchse 1 derart gestaltet sein, dass Abschnitte des zweiten axialen Bereichs 5 die Spannzange 9 umfassen.

Die Spannzange 9 kann Bestandteil des zweiten axialen Bereichs 5 sein. Vorzugsweise ist die Innenkontur des zweiten axialen Bereichs 5 zumindest teilweise, vorzugsweise zumindest zum größten Teil durch die Spannzange 9 gebildet. Wie aus den Figuren 1a und 2 hervorgeht, ist bei dem dort gezeigten Ausführungsbeispiel die Innenkontur des zweiten axialen Bereichs 5 überwiegend bzw. zum größten Teil durch die Spannzange 9 gebildet.

Der zweite axiale Bereich 5 kann sich unmittelbar an den ersten axialen Bereich 2 anschließen. Alternativ kann - wie in den Figuren 1a und 2 beispielhaft skizziert - entlang der Längsachse L betrachtet zwischen dem ersten axialen Bereich 2 und dem zweiten axialen Bereich 5 ein Zwischenabschnitt vorgesehen sein, in dem die Innenkontur der Führungsbuchse 1 in radialer Richtung durchgehend weiter als der Radius R von der Längsachse L entfernt ist.

Vorzugsweise schließt sich der erste axiale Bereich 2 insbesondere unmittelbar an eine Einführungsöffnung 6 für das Werkzeug an. Die Einführungsöffnung 6 ist dabei vorzugsweise als Teil des Kopfbereichs 8 gestaltet, insbesondere durch die Führungsbuchse 1. Vorzugsweise erstreckt sich - mit Bezug auf die Längsachse L - der zweite axiale Bereich 5 auf einer Seite des ersten axialen Bereichs 2, die der Einführungsöffnung 6 gegenüber liegt. Mit Bezug auf die Darstellungen der Figuren 1a und 2 erstreckt sich in diesem Sinn der zweite axiale Bereich 5 oberhalb des ersten axialen Bereichs 2 und die Einführungsöffnung 6 ist auf der Unterseite des ersten axialen Bereichs 2 gebildet.

Vorzugsweise ist die Erstreckung des ersten axialen Bereichs 2 entlang der Längsachse L - hier auch als Länge L1 des ersten axialen Bereichs 2 bezeichnet - kleiner als die Erstreckung des zweiten axialen Bereichs 5 entlang der Längsachse L - hier auch als Länge L2 des zweiten axialen Bereichs 5 bezeichnet. Beispielsweise kann vorgesehen sein, dass die Länge L1 des ersten axialen Bereichs 2 höchstens 20 % der Länge L2 des zweiten axialen Bereichs 5 beträgt. Dies ist vorteilhaft mit Bezug auf eine geeignete Führung des Werkzeugschafts.

Wie beispielhaft in Fig. 1b gezeigt, umfasst die Spannzange 9 weiterhin vorzugsweise einen Endabschnitt 91, in dem zumindest ein Spalt gebildet ist, der sich im Wesentlichen entlang der Längsachse L erstreckt. Weiterhin vorzugsweise weist das Behandlungsinstrument ein keilartiges Eingreifelement 98 auf, das derart parallel zur Längsachse L beweglich in dem Kopfbereich 8 gelagert ist, dass es mehr oder weniger weit in den zumindest einen Spalt eingreifen kann. Dabei ist die Spannzange 9 derart federelastisch gestaltet, dass sich ein radialer Durchmesser der Innenkontur des Endabschnitts 91 durch hin- und her bewegen des Eingreifelements 98 verringern oder vergrößern lässt. Auf diese Weise lässt sich durch ein Verringern des Durchmessers der Innenkontur des Endabschnitts 91 der Spannzange 9 die genannte Fixierung eines in die Führungsbuchse 1 eingesetzten rein zylindrischen Schaftabschnitts eines Werkzeugs erzielen, so dass das Werkzeug für einen Betrieb geeignet fixiert in der Führungsbuchse 1 angeordnet ist. Durch ein Vergrößern des Durchmessers lässt sich die Fixierung Lösen, so dass hierdurch das Werkzeug nach einem Gebrauch einfach, insbesondere manuell wieder aus der Führungsbuchse 1 heraus gezogen werden kann.

Vorzugsweise erstreckt sich mit Bezug auf die Längsachse L der Endabschnitt 91 der Spannzange 9 auf einer Seite des zweiten axialen Abschnitts 5, die dem ersten axialen Abschnitt 2 gegenüber liegt. Mit Bezug auf die Darstellung der Fig. 1b erstreckt sich der Endabschnitt 91 der Spannzange 9 in diesem Sinn oberhalb des zweiten axialen Abschnitts 5.

Weiterhin vorzugsweise weist die Führungsbuchse 1 außerdem eine Lager- bzw. Antriebshülse 7 auf, welche in dem Kopfbereich 8 drehbar gelagert ist, wobei die Antriebshülse 7 mit den Antriebsmitteln des Behandlungsinstruments zusammenwirkt, so dass eine besonders unmittelbare Drehmomentübertragung ermöglicht ist. Bei dem in Fig. 1a gezeigten Ausführungsbeispiel ist dabei der erste axiale Bereich 2 der Führungsbuchse 1 als integraler Bestandteil der Antriebshülse 7 gestaltet. So lässt sich erzielen, dass das Drehmoment besonders unmittelbar auf den Schaft des Werkzeugs übertragen wird.

Die Spannzange 9 ist vorzugsweise mit Bezug auf die Längsachse L auf einer Seite des ersten axialen Bereichs 2 angeordnet, die der Einführungsöffnung 6 gegenüber liegt. Mit Bezug auf die Darstellung der Fig. 1b ist die Spannzange 9 in diesem Sinn oberhalb des ersten axialen Bereichs 2 angeordnet, während die Einführungsöffnung 6 auf der Unterseite des ersten axialen Bereichs 2 gebildet ist.

Vorzugsweise besteht die Antriebshülse 7 aus einem härteren Material als die Spannzange 9. Mit anderen Worten besteht die Spannzange 9 vorzugsweise aus einem weicheren Material als die Antriebshülse 7. Dies ist vorteilhaft, weil sich hierdurch die Spannzange 9 besonders gut zur Fixierung des Werkzeugs eignet.

Bei der in den Figuren 1a und 1b skizzierten Ausgestaltung ist die Spannzange 9 als separates Bauteil gestaltet, das in die Antriebshülse 7 bzw. in die restliche Führungsbuchse 1 eingesetzt ist, insbesondere fest bzw. dauerhaft eingesetzt ist, beispielsweise eingepresst. Wie beispielsweise aus Fig. 1b hervorgeht, kann die Führungsbuchse 1 aus der Antriebshülse 7 und der Spannzange 9 bestehen.

Beispielsweise kann, wie aus Fig. 1a hervorgeht, der zweite axiale Bereich 5 zu einem Teil durch die Antriebshülse 7 gebildet sein und zu einem weiteren Teil durch die Spannzange 9. Mit Bezug auf die Fig. 2 ist ein unterer Anteil des zweiten axialen Bereichs 5 lediglich durch die Antriebshülse 7 gebildet und ein oberer Anteil des zweiten axialen Bereichs 5 radial innen durch die Spannzange 9 und radial außen durch die Antriebshülse 7.

Wie erwähnt, kann die Spannzange 9 in die Antriebshülse 7 eingesetzt sein, beispielsweise eingepresst. Insbesondere kann die Antriebshülse 7 hierzu eine radiale Ausnehmung 73 aufweisen, in die die Spanzange 9 entsprechend eingesetzt ist.

Hierbei ist die Gestaltung weiterhin vorzugsweise derart, dass die Antriebshülse 7 eine Schulterfläche 71 aufweist, die sich insbesondere in einer normal zur Längsachse L orientierten Ebene erstreckt, wobei diese Schulterfläche 71 eine Anlagefläche für die Spannzange 9 bildet.

Wie weiterhin aus den Figuren 1b und 2 hervorgeht, ist die Gestaltung dabei vorzugsweise derart, dass die Ausnehmung 73 mit Bezug auf die Längsachse L innerhalb eines Längsabschnitts λ eine insbesondere kreiszylindrische Kontaktfläche für einen unmittelbaren Kontakt zu der Spannzange 9 bildet, derart, dass im Betriebsfall die Drehmomentübertragung von der Antriebshülse 7 auf die Spannzange 9 über diese Kontaktfläche stattfindet. Vorzugsweise liegt hierfür - wie in Fig. 2 skizziert - der Längsabschnitt λ innerhalb des zweiten axialen Bereichs 5.

Vorzugsweise ist dabei die Gestaltung weiterhin derart, dass eine Trennung der Spannzange 9 von der Antriebshülse 7 im Rahmen des vorgesehenen Betriebsgebrauchs des Behandlungsinstruments nicht vorgesehen ist, also beispielsweise nicht zerstörungsfrei möglich ist.

Zwischen dem Endabschnitt 91 der Spannzange 9 und der Antriebshülse 7 ist vorzugsweise ein radialer Spalt 79 gebildet. Dies ist vorteilhaft mit Bezug auf die beschriebene Bewegung des Endabschnitts 91 der Spannzange 9 bei einer Vergrößerung des Durchmessers der Innenkontur des Endabschnitts 91.

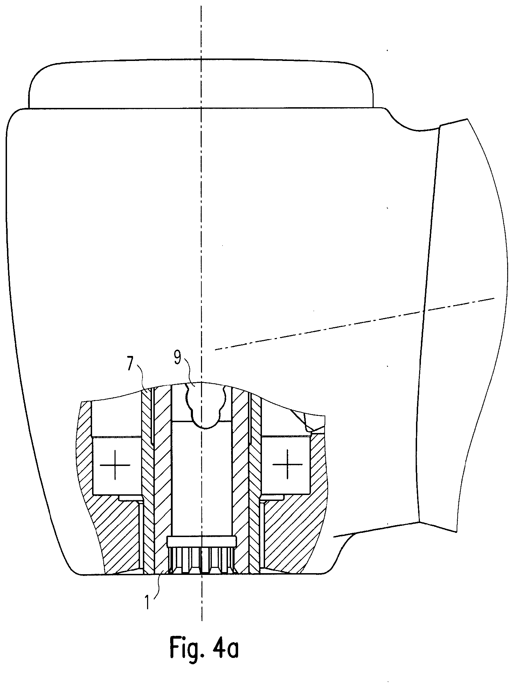

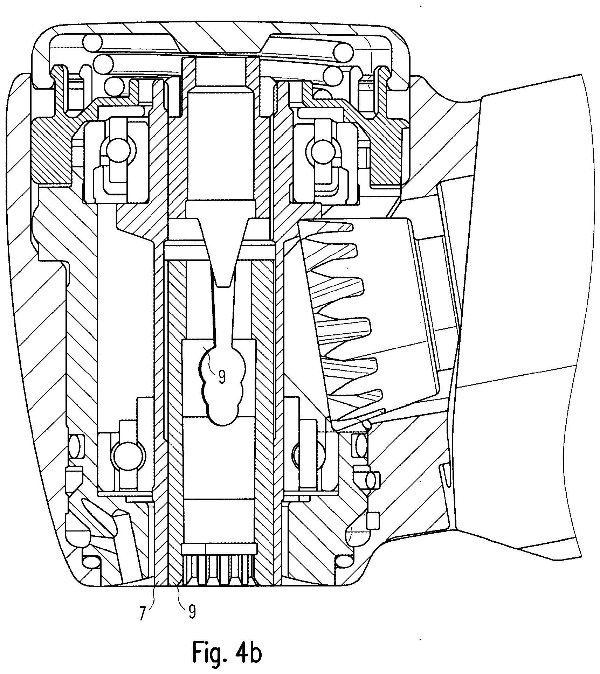

In Fig. 4a ist eine Variante der Ausgestaltung nach Fig. 1a gezeigt, wobei die Bezugszeichen analog zu oben gebraucht sind. Fig. 4b zeigt eine entsprechende Querschnittskizze. Bei dieser Ausgestaltung ist die Innenkontur des ersten axialen Bereichs 2 vollständig von der Spannzange 9 gebildet. Dementsprechend sind insbesondere die Flächenabschnitte 3 und die Ausnehmungen 4 vollständig von der Spannzange 9 gebildet. Mit anderen Worten ist hier der erste axiale Bereich 2 radial innen durch die Spannzange 9 gebildet und radial außen durch die Antriebshülse 7.

Vorzugsweise ist dabei die Gestaltung derart, dass die Spannzange 9 fest mit der Antriebshülse 7 verbunden ist, insbesondere derart, dass eine Trennung der Spannzange 9 von der Antriebshülse 7 im Rahmen des vorgesehenen Betriebsgebrauchs des Behandlungsinstruments nicht vorgesehen ist, also beispielsweise nicht zerstörungsfrei möglich ist. So lässt sich eine besonders geeignete Drehmomentübertragung zwischen der Antriebshülse 7 und der Spannzange 9 besonders geeignet erzielen.

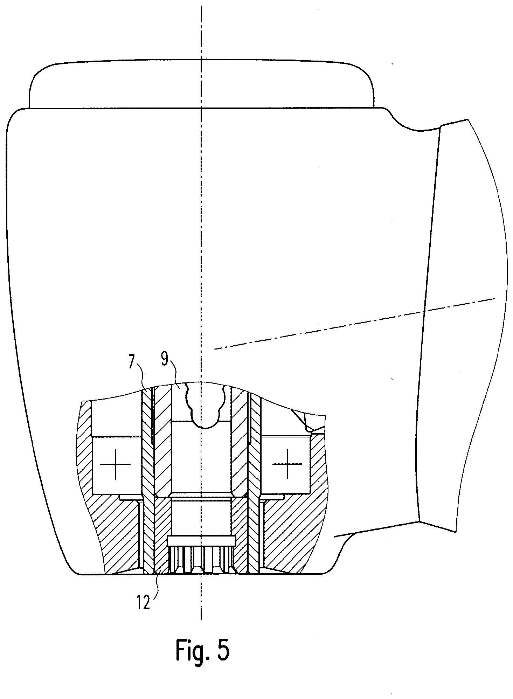

Fig. 5 zeigt eine weitere Variation. Hier ist der erste axiale Bereich 2 der Führungsbuchse 1 mit Hilfe eines separaten Bauteils 12 gestaltet, das in die Antriebshülse 7 entsprechend fest eingesetzt ist, wie beim zuletzt genannten Beispiel die Spannzange 9. Der erste axiale Bereich 2 ist hier radial innen durch das Bauteil 12 gebildet und radial außen durch die Antriebshülse 7.

Wie weiterhin aus Fig. 5 hervorgeht, ist hierbei die Spannzange 9 mit Bezug auf die Längsachse L auf einer Seite des separaten Bauteils 12 angeordnet, die der Einführungsöffnung 6 gegenüber liegt. Mit Bezug auf die Darstellung der Fig. 5 ist die Spannzange 9 in diesem Sinn oberhalb des Bauteils 12 angeordnet.

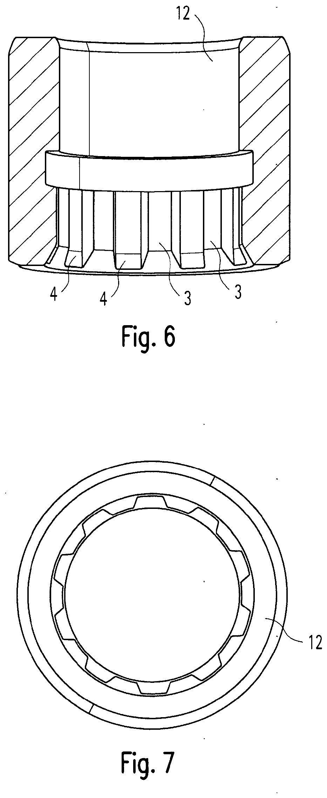

Fig. 6 zeigt eine perspektivische Schnittskizze des in Fig. 5 gezeigten Bauteils 12 und Fig. 7 eine Ansicht des Bauteils 12 entlang der Längsachse L. Dabei kann die Gestaltung derart sein, dass sich die Ausnehmungen 4 - wie skizziert - lediglich über einen Teilbereich der Längserstreckung des Bauteils 12 entlang der Längsachse L hinweg erstrecken. Über einen weiteren Teilbereich seiner Längserstreckung hinweg weist das Bauteil 12 analog zu oben eine vollständig kreiszylindrische Innenkontur mit dem Radius R auf. Dieser weitere Teilbereich dient dementsprechend wiederum zur Führung eines entsprechend kreiszylindrischen Werkzeugschafts.

Zwischen dem zuerst genannten Teilbereich mit den Ausnehmungen 4 dem zuletzt genannten weiteren Teilbereich mit der kreiszylindrischen Innenkontur kann ein Zwischenabschnitt gestaltet sein, dessen Begrenzungswände in radialer Richtung betrachtet durchgehend weiter als der Radius R von der Längsachse L entfernt sind.

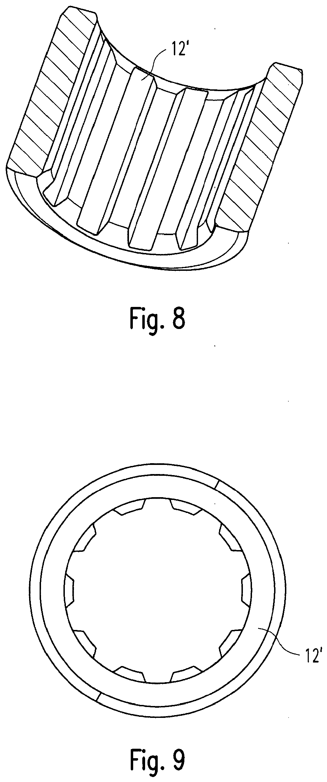

Fig. 8 zeigt eine perspektivische Schnittskizze zu einer Variation des in Fig. 5 gezeigten Bauteils, hier mit dem Bezugszeichen 12' versehen und Fig. 9 eine Ansicht dieses Bauteils 12' entlang der Längsachse. Bei dieser Ausgestaltung ist die innen gebildete Struktur sozusagen durchgehend gestaltet, sie erstreckt sich also über die gesamte Längserstreckung des Bauteils 12' hinweg.

Im Übrigen kann das Spannsystem wie an sich bekannt gestaltet sein. Beispielsweise kann zum Lösen des Werkzeugs bzw. für einen Werkzeugwechsel eine entsprechende an sich bekannte Druckknopfbetätigung vorgesehen sein.

Die Antriebshülse 7 kann aus einem härtbaren Edelstahl bestehen, insbesondere mit einem Härtegrad, der größer als 450 HV ist. Die Spannzange 9 kann aus einem härtbarem Edelstahl bestehen. Die Spannzange 9 kann derart gestaltet sein, dass sie eine Spannkraft von mehr als 22 N aufweist. Das Bauteil 12 kann beispielsweise aus einem härtbaren Edelstahl bestehen oder beispielsweise aus einem Hartmetall.

Mit einem anmeldungsgemäßen Behandlungsinstrument lassen sich insbesondere die folgenden Vorteile erzielen:
- Es können auftretende Drehmomente abgefangen werden und somit kann Schlupf vermieden werden.
- Durch die Verhinderung von Schlupf lässt sich insbesondere eine zusätzliche Erwärmung durch Reibung an der formschlüssigen Verbindung vermeiden.
- Das Spannsystem wird geschont und kann so eine sichere Funktion gewährleisten.
- Die Werkzeugschäfte werden geschont und das Problem klemmender bzw. nur schwer zu entnehmender Werkzeuge lässt sich praktisch vermeiden.
- Das Behandlungsinstrument lässt sich vorteilhaft als Schnelllaufwinkelstück oder als Turbine gestalten.
- Für das Spannsystem lässt sich eine besonders lange Lebensdauer erzielen, insbesondere im Vergleich zu Spannsystemen vergleichbarer Serien-Instrumente.
- Es können viele Standardkomponenten eines bekannten Kopfgehäuses weiterhin verwendet werden.
- Trotz der Möglichkeit der formschlüssigen Verbindung können alternativ weiterhin Standardwerkzeuge nach DIN EN ISO 1797 verwendet werden.
- Dabei ist weiterhin ein gewohntes, schnelles und (nahezu) stellungsunabhängiges Einsetzen des Werkzeugs in das Spannsystem ermöglicht.
- Ein Werkzeugwechsel ist über den gewohnten Druckknopf möglich.
- Das Werkzeug kann in der Antriebshülse bzw. der Führungsbuchse sicher und eng geführt werden; dies ist vorteilhaft mit Bezug auf einen geringen Bohrerschlag und eine geeignete Laufruhe.

## Patentansprüche

1. Zahnärztliches Behandlungsinstrument zum Betreiben eines Rotationswerkzeugs, wobei das Behandlungsinstrument einen zur Kopplung mit einem Schaft des Rotationswerkzeugs vorgesehenen Kopftrieb mit einer hülsenartigen Führungsbuchse (1) aufweist,
wobei die Führungsbuchse (1) eine Spannzange (9) zur Fixierung des in die Führungsbuchse (1) eingesetzten Rotationswerkzeugs aufweist,
und wobei die Führungsbuchse (1) in zumindest einem ersten axialen Bereich (2) Flächenabschnitte (3) aufweist, welche Teile einer Zylinderform bilden, wobei von diesen Flächenabschnitten (3) radial nach außen weisende Ausnehmungen (4) vorgesehen sind, welche der Innenkontur der Führungsbuchse (1) eine von einer rotationssymmetrischen Form abweichende Form verleihen.

2. Zahnärztliches Behandlungsinstrument nach Anspruch 1,
**dadurch gekennzeichnet,**
**dass** die Führungsbuchse (1) einen zweiten axialen Bereich (5) aufweist, dessen Innenkontur eine vollständige Zylinderform bildet, welche hinsichtlich ihres Durchmessers (2R) identisch zu der durch die Flächenabschnitte (3) des ersten axialen Bereichs (2) gebildeten Zylinderform ist,
wobei vorzugsweise Abschnitte des zweiten axialen Bereichs (5) die Spannzange (9) umfassen.

3. Zahnärztliches Behandlungsinstrument nach Anspruch 2,
**dadurch gekennzeichnet,**
**dass** die Innenkontur des zweiten axialen Bereichs (5) teilweise durch die Spannzange (9) gebildet ist.

4. Zahnärztliches Behandlungsinstrument nach Anspruch 2 oder 3,
**dadurch gekennzeichnet,**
**dass** sich der erste axiale Bereich (2) an eine Einführungsöffnung (6) für das Werkzeug anschließt.

5. Zahnärztliches Behandlungsinstrument nach einem der Ansprüche 2 bis 4,
**dadurch gekennzeichnet,**
**dass** die Länge (L1) des ersten axialen Bereichs (2) kleiner ist als die Länge (L2) des zweiten axialen Bereichs (5),
wobei vorzugsweise die Länge (L1) des ersten axialen Bereichs (2) höchstens 20% der Länge (L2) des zweiten axialen Bereichs (5) beträgt.

6. Zahnärztliches Behandlungsinstrument nach einem der Ansprüche 1 bis 5,
**dadurch gekennzeichnet,**
**dass** die Führungsbuchse (1) eine Lager- bzw. Antriebshülse (7) aufweist, welche in einem Kopfbereich (8) des Behandlungsinstruments drehbar gelagert ist und mit Antriebsmitteln des Behandlungsinstruments zusammenwirkt.

7. Zahnärztliches Behandlungsinstrument nach Anspruch 6,
**dadurch gekennzeichnet,**
**dass** der erste axiale Bereich (2) der Führungsbuchse (1) als integraler Bestandteil der Antriebshülse (7) gestaltet ist.

8. Zahnärztliches Behandlungsinstrument nach Anspruch 6 oder 7,
**dadurch gekennzeichnet,**
**dass** die Antriebshülse (7) aus einem härteren Material besteht als die Spannzange (9).

9. Zahnärztliches Behandlungsinstrument nach einem der vorherigen Ansprüche,
**dadurch gekennzeichnet,**
**dass** die Ausnehmungen (4) längliche Kerben bilden, welche sich axial erstrecken, wobei vorzugsweise mehrere Kerben vorgesehen sind, welche am Umfang der Rotationsform verteilt, vorzugsweise gleichmäßig verteilt angeordnet sind.

10. Zahnärztliches Behandlungsinstrument nach einem der vorherigen Ansprüche,
**dadurch gekennzeichnet,**
**dass** dieses Antriebsmittel zum Übertragen einer Rotation auf ein in die Führungsbuchse (1) aufgenommenes Rotationswerkzeug aufweist.

11. Zahnärztliches Behandlungsinstrument nach Anspruch 10,
**dadurch gekennzeichnet,**
**dass** die Antriebsmittel einen Elektromotor oder eine Turbine umfassen.

12. Zahnärztliches Behandlungsinstrument nach einem der Ansprüche 1 bis 11 mit den im Anspruch 6 genannten Merkmalen,
**dadurch gekennzeichnet,**
**dass** die Spannzange (9) in eine radiale Ausnehmung (73) der Antriebshülse (7) eingesetzt ist.

13. Zahnärztliches Behandlungsinstrument nach Anspruch 12 mit den in den Ansprüchen 4 und 7 genannten Merkmalen,
**dadurch gekennzeichnet,**
**dass** die Spannzange (9) mit Bezug auf die Längsachse (L) auf einer Seite des ersten axialen Bereichs (2) angeordnet ist, die der Einführungsöffnung (6) gegenüber liegt.

14. Zahnärztliches Behandlungsinstrument nach Anspruch 6,
**dadurch gekennzeichnet,**
**dass** der erste axiale Bereich (2) der Führungsbuchse (1) mit Hilfe eines separaten Bauteils (12) gestaltet ist, das in die Antriebshülse (7) eingesetzt ist.

15. Zahnärztliches Behandlungsinstrument nach Anspruch 13 mit den im Anspruch 4 genannten Merkmalen,
**dadurch gekennzeichnet,**
**dass** die Spannzange (9) mit Bezug auf die Längsachse (L) auf einer Seite des separaten Bauteils (12) angeordnet ist, die der Einführungsöffnung (6) gegenüber liegt.

## Claims

1. Dental treatment instrument for operating a rotary tool,
wherein the treatment instrument has a head drive, which is provided for coupling to a shank of the rotary tool and which has a sleeve-like guide bushing (1),
wherein the guide bushing (1) has a collet chuck (9) for fixing the rotary tool inserted into the guide bushing (1),
and wherein the guide bushing (1) has, in at least a first axial region (2), surface portions (3) which form parts of a cylindrical form, wherein recesses (4) are provided which face radially outwards from these surface portions (3) and which give the inner contour of the guide bushing (1) a form that deviates from a rotationally symmetrical form.

2. Dental treatment instrument according to Claim 1, **characterized in that** the guide bushing (1) has a second axial region (5), of which the inner contour forms a complete cylindrical form which, in terms of its diameter (2R), is identical to the cylindrical form formed by the surface portions (3) of the first axial region (2),
wherein portions of the second axial region (5) preferably comprise the collet chuck (9).

3. Dental treatment instrument according to Claim 2, **characterized in that** the inner contour of the second axial region (5) is partially formed by the collet chuck (9).

4. Dental treatment instrument according to Claim 2 or 3, **characterized in that** the first axial region (2) adjoins an insertion opening (6) for the tool.

5. Dental treatment instrument according to one of Claims 2 to 4, **characterized in that** the length (L1) of the first axial region (2) is shorter than the length (L2) of the second axial region (5), wherein the length (L1) of the first axial region (2) is preferably at most 20% of the length (L2) of the second axial region (5).

6. Dental treatment instrument according to one of Claims 1 to 5, **characterized in that** the guide bushing (1) has a bearing sleeve or drive sleeve (7), which is rotatably mounted in a head region (8) of the treatment instrument and cooperates with drive means of the treatment instrument.

7. Dental treatment instrument according to Claim 6, **characterized in that** the first axial region (2) of the guide bushing (1) is configured as an integral component part of the drive sleeve (7).

8. Dental treatment instrument according to Claim 6 or 7, **characterized in that** the drive sleeve (7) is made of a harder material than the collet chuck (9) .

9. Dental treatment instrument according to one of the preceding claims, **characterized in that** the recesses (4) form elongate notches that extend axially, wherein a plurality of notches are preferably provided which are arranged distributed, preferably uniformly distributed, at the circumference of the rotational form.

10. Dental treatment instrument according to one of the preceding claims, **characterized in that** it has drive means for transmitting a rotation to a rotary tool received in the guide bushing (1).

11. Dental treatment instrument according to Claim 10, **characterized in that** the drive means comprise an electric motor or a turbine.

12. Dental treatment instrument according to one of Claims 1 to 11, having the features set forth in Claim 6, **characterized in that** the collet chuck (9) is inserted into a radial recess (73) of the drive sleeve (7).

13. Dental treatment instrument according to Claim 12, having the features set forth in Claims 4 and 7, **characterized in that** the collet chuck (9), with respect to the longitudinal axis (L), is arranged on a side of the first axial region (2) lying opposite the insertion opening (6).

14. Dental treatment instrument according to Claim 6, **characterized in that** the first axial region (2) of the guide bushing (1) is configured with the aid of a separate component (12), which is inserted into the drive sleeve (7).

15. Dental treatment instrument according to Claim 13, having the features set forth in Claim 4, **characterized in that** the collet chuck (9), with respect to the longitudinal axis (L), is arranged on a side of the separate component (12) lying opposite the insertion opening (6).

## Revendications

1. Instrument dentaire de traitement pour le fonctionnement d'un outil rotatif, dans lequel l'instrument de traitement présente un moteur de tête prévu pour le couplage à une tige de l'outil rotatif avec une douille de guidage (1) de type manchon,
dans lequel la douille de guidage (1) présente une pince de serrage (9) pour la fixation de l'outil rotatif inséré dans la douille de guidage (1),
et dans lequel la douille de guidage (1) présente dans au moins une première zone axiale (2) des sections de surface (3), lesquelles forment des parties d'une forme cylindrique, dans lequel des évidements (4) dirigés radialement vers l'extérieur de ces sections de surface (3) sont prévus, lesquels confèrent au contour intérieur de la douille de guidage (1) une forme divergente d'une forme à symétrie de révolution.

2. Instrument dentaire de traitement selon la revendication 1,
**caractérisé en ce**
**que** la douille de guidage (1) présente une deuxième zone axiale (5), dont le contour intérieur forme une forme cylindrique complète, laquelle est identique en ce qui concerne son diamètre (2R) à la forme cylindrique formée par les sections de surface (3) de la première zone axiale (2),
dans lequel de préférence des sections de la deuxième zone axiale (5) comprennent la pince de serrage (9).

3. Instrument dentaire de traitement selon la revendication 2,
**caractérisé en ce**
**que** le contour intérieur de la deuxième zone axiale (5) est formé en partie par la pince de serrage (9).

4. Instrument dentaire de traitement selon la revendication 2 ou 3,
**caractérisé en ce**
**que** la première zone axiale (2) se raccorde à une ouverture d'introduction (6) pour l'outil.

5. Instrument dentaire de traitement selon l'une quelconque des revendications 2 à 4,
**caractérisé en ce**
**que** la longueur (L1) de la première zone axiale (2) est plus petite que la longueur (L2) de la deuxième zone axiale (5),
dans lequel de préférence la longueur (L1) de la première zone axiale (2) est au maximum 20 % de la longueur (L2) de la deuxième zone axiale (5).

6. Instrument dentaire de traitement selon l'une quelconque des revendications 1 à 5,
**caractérisé en ce**
**que** la douille de guidage (1) présente un manchon de stockage ou d'entraînement (7), lequel est logé de manière rotative dans une zone de tête (8) de l'instrument de traitement et coopère avec des moyens d'entraînement de l'instrument de traitement.

7. Instrument dentaire de traitement selon la revendication 6,
**caractérisé en ce**
**que** la première zone axiale (2) de la douille de guidage (1) est configurée en tant que partie intégrante du manchon d'entraînement (7).

8. Instrument dentaire de traitement selon la revendication 6 ou 7,
**caractérisé en ce**
**que** le manchon d'entraînement (7) est constitué d'un matériau plus dur que la pince de serrage (9).

9. Instrument dentaire de traitement selon l'une quelconque des revendications précédentes,
**caractérisé en ce**
**que** les évidements (4) forment des encoches oblongues, lesquelles s'étendent axialement, dans lequel de préférence plusieurs encoches sont prévues, lesquelles sont réparties sur la circonférence de la forme rotative, de préférence réparties uniformément.

10. Instrument dentaire de traitement selon l'une quelconque des revendications précédentes,
**caractérisé en ce**
**que** celui-ci présente des moyens d'entraînement pour la transmission d'une rotation à un outil rotatif reçu dans la douille de guidage (1).

11. Instrument dentaire de traitement selon la revendication 10,
**caractérisé en ce**
**que** les moyens d'entraînement comprennent un moteur électrique ou une turbine.

12. Instrument dentaire de traitement selon l'une quelconque des revendications 1 à 11 avec les caractéristiques mentionnées dans la revendication 6,
**caractérisé en ce**
**que** la pince de serrage (9) est insérée dans un évidement radial (73) du manchon d'entraînement (7).

13. Instrument dentaire de traitement selon la revendication 12 avec les caractéristiques mentionnées dans les revendications 4 et 7,
**caractérisé en ce**
**que** la pince de serrage (9) est agencée par rapport à l'axe longitudinal (L) d'un côté de la première zone axiale (2), qui se trouve en face de l'ouverture d'introduction (6).

14. Instrument dentaire de traitement selon la revendication 6,
**caractérisé en ce**
**que** la première zone axiale (2) de la douille de guidage (1) est configurée à l'aide d'un composant séparé (12), qui est inséré dans la douille d'entraînement (7).

15. Instrument dentaire de traitement selon la revendication 13 avec les caractéristiques mentionnées dans la revendication 4,
**caractérisé en ce**
**que** la pince de serrage (9) est agencée par rapport à l'axe longitudinal (L) d'un côté du composant séparé (12), qui se trouve en face de l'ouverture d'introduction (6).
